# EUROPÄISCHE PATENTSCHRIFT

(11) **EP 0 438 499 B1**
(45) Veröffentlichungstag und Bekanntmachung des Hinweises auf die Patenterteilung: **08.06.1994**
(21) Anmeldenummer: 89911979.6
(22) Anmeldetag: 11.10.1989
(51) Int. Cl.: A61L 27/00

(54) **VERFAHREN ZUR HERSTELLUNG VON RINDER-PERICARD-MATERIALIEN UND IHRE VERWENDUNG**
PROCESS FOR PRODUCING BOVINE PERICARDIUM MATERIAL AND USE THEREOF
PROCEDE POUR LA FABRICATION DE MATERIAUX PERICARDIQUES DE B UF ET LEUR UTILISATION

(30) Priorität: 15.10.1988 DE 3835237
(43) Veröffentlichungstag der Anmeldung: 31.07.1991
(73) Patentinhaber: B. Braun Melsungen AG, 34209 Melsungen (DE)
(72) Erfinder: SCHOLL, Edmund, D-3508 Melsungen (DE); WALDERT, Helmut, D-3508 Melsungen (DE); BEYER, Helmut, D-3507 Baunatal (DE)
(74) Vertreter: von Kreisler, Alek, Dipl.-Chem.
(86) Internationale Anmeldenummer: EP8901202
(87) Internationale Veröffentlichungsnummer: WO9003811

(56) Entgegenhaltungen:
- EP-A- 0 172 716
- GB-A- 2 196 008
- US-A- 4 776 853

## Beschreibung

Die Vorliegende Erfindung betrifft ein Verfahren zur Herstellung von Rinder-Pericard-Materialien, die eine ausgezeichnete Biokompatibilität sowie eine erhöhte Stabilität besitzen, und die Verwendung der so hergestellten Materialien als Transplantate oder Implantate in der Humanmedizin und Veterinärmedizin.

Seit Anfang dieses Jahrhunderts versucht man in der Chirurgie größere Gewebsdefekte mit Hilfe von Transplantaten aus kollagenem Bindegewebe zu decken (Marchant, 1901; Kleinschmidt, 1914). Seit 1954 steht humane Dura mater als homologes Transplantat in lyophilisierter Form, seit 1973 in Form eines lösungsmittelgetrockneten Produkts zur Verfügung. In vielen Fällen hat sich Humandura dank ihrer guten Gewebeverträglichkeit, des Fehlens immunologischer Reaktionen und nicht zuletzt wegen der hohen Reißfestigkeit bewährt, so daß dieses Biomaterial auch heute noch mit Erfolg eingesetzt wird. Leider steht Dura mater als Human-Transplantat aber nur in sehr begrenztem Umfang zur Verfügung und kann daher den Bedarf seitens der Chirurgie bei weitem nicht decken. Auch gibt es einige Indikationen, zum Beispiel in der Neurochirurgie und im HNO-Bereich, bei denen sich Dura-Transplantate häufig als etwas zu steif und von der Dicke her nicht optimal erwiesen haben.

Die DE-OS 30 34 273 betrifft ein Verfahren zur Herstellung von Kollagen, das dadurch gekennzeichnet ist, daß natürliches, unlösliches Collagen mit einer wäßrigen Lösung eines Alkalisulfats und/oder Alkalihydroxids behandelt wird, daß man das fettfreie Kollagen mit einer wäßrigen Lösung, die ein Alkalisulfat enthält, behandelt und gegebenfalls mit destilliertem Wasser wäscht, das Kollagen in einer wäßrigen Lösung löst, der Lösung gegebenenfalls eine kleine Menge einer biologisch wirksamen Substanz, wie ein Antibiotikum, ein Hormon oder ein Spermizid, zusetzt, diese Lösung einfriert und das Produkt unter Vakuum trocknet.

Allerdings nimmt diese Offenlegungsschrift den Gegenstand der vorliegenden Erfindung weder in allen Merkmalen vorweg noch legt sie diesen für den Fachmann nahe.

Das US-Patent 4 006 083 beschreibt ein steriles, in der Chirurgie einsetzbares Kollagenprodukt mit einer Filz- oder Webpelzstruktur, welches eine hämostatische Wirkung ausübt, eine hohe Absorptionskapazität für Körperflüssigkeiten besitzt, die Zellregeneration anregt, ein hohes Resorptionsvermögen aufweist, im wesentlichen nicht von Körpergeweben abgestoßen wird und das optimale mechanische Eigenschaften besitzt, was dieses Material dazu befähigt, bei der Versorgung von Hautverletzungen oder in Hautverletzungen oder in Knochenhöhlungen eingesetzt zu werden.

Allerdings nimmt die Lehre dieses US-Patents den Gegenstand der vorliegenden Erfindung weder in allen Merkmalen vorweg, noch legt sie diesen nahe.

Die EP-A-0 172 716 beschreibt ein Herstellungsverfahren für Implantate aus Rinderpericard, das zunächst mit einer gepufferten, ein amphoteres Tensid enthaltenden Salzlösung behandelt wird, daraufhin mit einer gepufferten Salzlösung gewaschen und schließlich mittels Glutaraldehydlösung gegerbt wird.

Die US-A-4 776 853 beschreibt ein Verfahren zur Herstellung eines biologischen Materials zur Implantation, beispielsweise aus Rinderpericard, das folgende Verfahrensschritte aufweist:
1. Extraktion des aus tierischen Kadavern gewonnenen Gewebes mittels einer hypotonischen schwach basischen Pufferlösung, die Antibiotika und proteolytische Inibitoren enthält,
2. Extraktion der Gewebeproben mittels einer schwach basischen Lösung mit einer hohen Salzkonzentration, welche ein nicht ionisches Tensid, ein Antibiotikum und einen Protease-Inibitor aufweist,
3. Behandlung der Gewebeprobe mit Enzymen in einer gepufferten Salzlösung, wobei die Enzyme aus gereinigter proteasefreier Deoxyribonuklease und Ribonuklease bestehen,
4. Extraktion der Gewebeproben mit einem anionischen Detergens bei schwach basischem pH und
5. Aufbewahrung der Gewebeproben in einer physiologischen Pufferlösung.

Das US-Patent 4 502 159 beschreibt die Herstellung einer röhrenförmigen Prothese, wie beispielsweise ein Gefäß oder eine Harnleiterprothese. Diese wird durch Zusammennähen der entgegengesetzten Ecken eines Pericardgewebes mit einem biokompatiblen Faden hergestellt. Es wird eine exakte Beschreibung beim Vorgehen des Nähens angegeben. Vorzugsweise verwendet man Pericard vom Rind, das ähnliche Dehnbarkeiten wie humane Gewebe besitzt. Das röhrenförmige Gewebe wird nach dem Vernähen für 7 Tage in einer 0,5 %igen Glutardialdehyd-Lösung gegerbt. Die glatte Seite des Pericardgewebes befindet sich im Lumen, die Basalmembranen auf der Oberfläche. Sterilisation erfolgt durch Konservierung in Formalin bzw. mit Strahlensterilisation einer das Pericard enthaltenden physiologischen Kochsalzlösung.

Allerdings gibt dieses US-Patent keine Hinweise über die Isolierung, Reinigung bzw. die physikalischen und chemischen Eigenschaften der so hergestellten röhrenförmigen Prothese, so daß der Gegenstand des US-Patents den Gegenstand der vorliegenden Erfindung nicht nahelegen kann.

Aus diesen Gegebenheiten des Standes der Technik resultierte zwangsläufig der Wunsch, ein praktisch unbegrenzt verfügbares Transplantat bzw. Implantat bereitzustellen, das möglichst alle positiven Eigenschaften der Dura mater in sich vereinigt und darüber hinaus noch gewisse Vorteile bei der Handhabung bietet. Zusätzlich sollte gewährleistet sein, daß keine für den Menschen gefährlichen Keime (HIV-Viren, Hepatitis-Viren etc.) durch das Rohmaterial eingeschleppt werden konnten, wie dies bei humanen Transplantaten grundsätzlich möglich ist.

Unter Berücksichtigung aller produktrelevanten Parameter wurde in bovinem Pericard - im folgenden auch Pericard genannt - ein biologisches Material gefunden, das als Implantat zur Deckung humaner Gewebsdefekte in nahezu optimaler Weise den Anforderungen genügte.

Die Erfindung stellt sich die Aufgabe, ein Verfahren zur Herstellung von Rinder-Pericard-Materialien zu schaffen, bei dem die biologische Kompatibilität und Stabilität des hierbei erhaltenen Erzeugnisses verbessert wird. Weitere Aufgabe der vorliegenden Erfindung ist die Bereitstellung neuer Verwendungsmöglichkeiten für dieses Erzeugnis.

Bei experimentellen Untersuchungen wurde nun überraschend gefunden, daß durch ein modifiziertes Herstellungsverfahren die Resistenz der im Rinder-Pericard enthaltenen Skleroproteine gegen biologischen Abbau sowie ihre Biokompatibilität auch ohne Aldehyd-Gerbung mittels Formaldehyd oder Glutardialdehyd - wie dies in US-A 4.502.159, WO 84/04669 sowie US-A 4.456.589 beschrieben ist - gesteigert werden kann.

Die vorliegende Erfindung betrifft somit ein Verfahren zur Herstellung von Rinder-Pericard-Material durch Aufarbeitung von rohem Rinder-Pericard-Gewebe durch eine naßchemische Aufbereitung, oxidative Bleichung, Auswaschen, Entfetten, Trocknen und Sterilisieren, das dadurch gekennzeichnet ist, daß die naßchemische Aufbereitung des Rinder-Pericard-Gewebes folgende Schritte umfaßt:
a) Wässern des Rinder-Pericard-Gewebes,
b) mechanische Entfernung von Fettgewebe und Basalmembranen von der Oberfläche des Rinder-Pericard-Gewebes,
c) Behandlung des Rinder-Pericard-Gewebes mit einer verdünnten, wäßrigen, basischen Lösung von Natriumhydroxid, Kaliumhydroxid, Lithiumhydroxid und/oder Natriumcarbonat über einen Zeitraum von bis zu 16 Stunden,
d) Spülen des Rinder-Pericard-Gewebes mit entmineralisiertem Wasser zur Entfernung von restlicher Base,
e) Behandlung des Rinder-Pericard-Gewebes mit verdünnter wäßriger Natriumchloridlösung,
f) Spülen des Rinder-Pericard-Gewebes mit entmineralisiertem Wasser zur Entfernung von Natriumchloridresten,
g) Behandlung des Rinder-Pericard-Gewebes mit einem Komplexierungsmittel,
h) Spülen des Rinder-Pericard-Gewebes mit entmineralisiertem Wasser zur Entfernung von Komplexierungsmittelresten,
i) Behandlung des Rinder-Pericard-Gewebes mit einem sauren Puffersystem und
j) Spülen des Rinder-Pericard-Gewebes mit entmineralisiertem Wasser zur Entfernung von sauren Puffersystemresten, und daß man das Entfetten und Trocknen durch Aceton-Behandlung bei Raumtemperatur, Soxhlet-Extraktion mit Aceton, Rehydratisierung und Gefriertrockung durchführt.

Nach einer bevorzugten Ausführungsform der vorliegenden Erfindung wiederholt man im Anschluß an Verfahrensschritt d) vor Verfahrensschritt e) die Maßnahme nach Merkmal b).

Obgleich die vorstehend beschriebenen Maßnahmen im naßchemischen Aufbereitungsschritt ohne weiteres auch oberhalb von Raumtemperatur oder unterhalb von Raumtemperatur durchgeführt werden können, so ist es doch im Sinne der vorliegenden Erfindung bevorzugt, sämtliche naßchemischen Verfahrensschritte bei Raumtemperatur, also zwischen 15 °C und 25 °C durchzuführen.

Vorzugsweise wird die Behandlung im Verfahrensschritt c) in einer verdünnten wäßrigen basischen Lösung von Natriumhydroxid, Kaliumhydroxid, Lithiumhydroxid und/oder Natriumcarbonat durchgeführt. Besonders bevorzugt ist die Verwendung einer 2 bis 2,5 Gew.-%igen wäßrigen Natriumhydroxidlösung, welche im allgemeinen über einen Zeitraum bis zu 16 Stunden auf das Rinder-Pericard-Gewebe einwirken soll, wobei man pro 100 g Rinder-Pericard-Gewebe 0,50 bis 1,00 l der verdünnten basischen Lösung verwendet. Hierdurch wird einerseits ein guter Reinigungseffekt erreicht, der eine möglichst vollständige Inaktivierung von Enzymen und potentiellen Keimen einschließt, andererseits aber auch eine Schädigung des Kollagen-haltigen Gewebes vermieden.

Nach der Behandlung mit der verdünnten, wäßrigen, basischen Lösung ist das Pericard-Gewebe im allgemeinen stark auf das 2-bis 5fache des Ursprungsvolumens aufgequollen.

Vorzugsweise wird die Behandlung im Verfahrensschritt e) mit einer 10 bis 11 Gew.-%igen Natriumchloridlösung ausgeführt, wodurch ein für die weitere Verarbeitung günstiger Quellzustand des Kollagens eingestellt werden kann. Niedrigere Konzentrationen an Natriumchloridlösungen bewirken demgegenüber eine nur unzureichende Entquellung des nach der alkalischen Behandlung stark gequollenen Kollagens. Höhere Konzentrationen an Natriumchloridlösung würden das Rinder-Pericard-Material nur unnötig mit Salzresten anreichern und sind schon deshalb nicht sinnvoll.

Vorzugsweise wird die Behandlung im Verfahrensschritt g) mit einem dem Fachmann bekannten Komplexierungsmittel für die Komplexierung mehrwertiger Metallionen, die zum Teil proteingebunden vorliegen, durchgeführt. Als besonders bevorzugt hat sich in diesem Falle eine Dinatrium-EDTA-Lösung erwiesen, welche beispielsweise in einer Konzentration von 0,3 bis 0,5 Gew.-% vorliegt. Dieses Komplexierungsreagenz muß, um seine komplexierende Wirkung voll entfalten zu können, auf einen basischen pH-Wert von mehr als 11 eingestellt werden. Die Behandlung mit dem Komplexierungsmittel sollte im allgemeinen über einen Zeitraum von 0,5 bis 2 h durchgeführt werden.

Vorzugsweise setzt man die Spülung im Verrahrensschritt h) so lange fort, bis man einen schwach-alkalischen pH-Wert von maximal 8,5 erreicht hat.

Vorzugsweise setzt man für die Behandlung in Verfahrensschritt i) als saures Puffersystem ein Acetat-Puffersystem mit einem pH-Wert von 4,5 bis 6,0 ein.

Vorzugsweise wird der nach der naßchemischen Aufbereitung folgende Schritt der oxidativen Bleichung in der Art ausgeführt, daß man das Rinder-Pericard-Gewebe mit einer Wasserstoffperoxid-haltigen wäßrigen Lösung mit einem Gehalt von weniger als 2 Gew.-%, insbesondere um 1,5 Gew.-% vornimmt. Diese Bleichung mittels Wasserstoffperoxid dient dem Zweck, noch vorhandene Begleitstoffe des Kollagens oxidativ zu zerstören, ohne das Kollagen selbst anzugreifen. Es hat sich gezeigt, daß Konzentrationen von Wasserstoffperoxid oberhalb 2,0 Gew.-% im Sinne der Erfindung nicht geeignet sind, da diese bereits eine merkliche Schädigung des Kollagen-haltigen Rinder-Pericard-Gewebes bewirken. Die Wasserstoff-Peroxid-Behandlung wird im allgemeinen über einen Zeitraum von 0,5 bis 2 Stunden durchgeführt, wobei man pro 100 g Rinder-Pericard-Gewebe 0,5 bis 1,0 l Wasserstoffperoxidlösung verwendet.

Im Anschluß an diese oxidative Bleichung werden Wasserstoffperoxidreste durch erschöpfendes Auswaschen mit Wasser in an sich bekannter Weise durchgeführt.

Das Entfetten und Trocknen des Rinder-Pericard-Gewebes erfolgt in der Weise, daß die Stücke zunächst mit einer ausreichenden Menge an Aceton bedeckt werden, und über einen Zeitraum von 12 bis 24 h dieses Lösungsmittel 2 bis 6 mal erneuert wird. Das so entwässerte Rinder-Pericard-Gewebe wird in eine Soxhlet-Apparatur überführt und in einer erschöpfenden Extraktion restliche Wassermengen und Fettreste entfernt. Nach der Extraktion werden die Rinder-Pericard-Stücke an der Luft getrocknet und anschließend in entmineralisiertem Wasser rehydratisiert (Quellung).

Anschließend wird das Rinder-Pericard-Gewebe in einer automatisch gesteuerten Gefriertrocknungsanlage gefriergetrocknet und sterilisiert.

Das vorstehend beschriebene erfindungsgemäße Herstellungsverfahren ist in besonderer Art dazu geeignet, um ein im medizinischen Bereich einsetzbares Produkt gleichbleibender Qualität zu erzeugen.

Das erfindungsgemäß erhaltene Rinder-Pericard kann als Translantat oder Implantat auf den verschiedensten Gebieten der Veterinärmedizin oder Humanmedizin, die dem Fachmann wohlbekannt sind, eingesetzt werden. Verwiesen ist in diesem Zusammenhang auf die Firmenbroschüre der Fa. B. Braun Melsungen AG "Lyodura^{R} zum homöoplastischen Ersatz von Körperstrukturen" aus dem Jahre 1978 und die darin genannten sehr zahlreichen Literaturstellen.

### Beurteilung der Reißfestigkeit im subkutanen Implantationstest

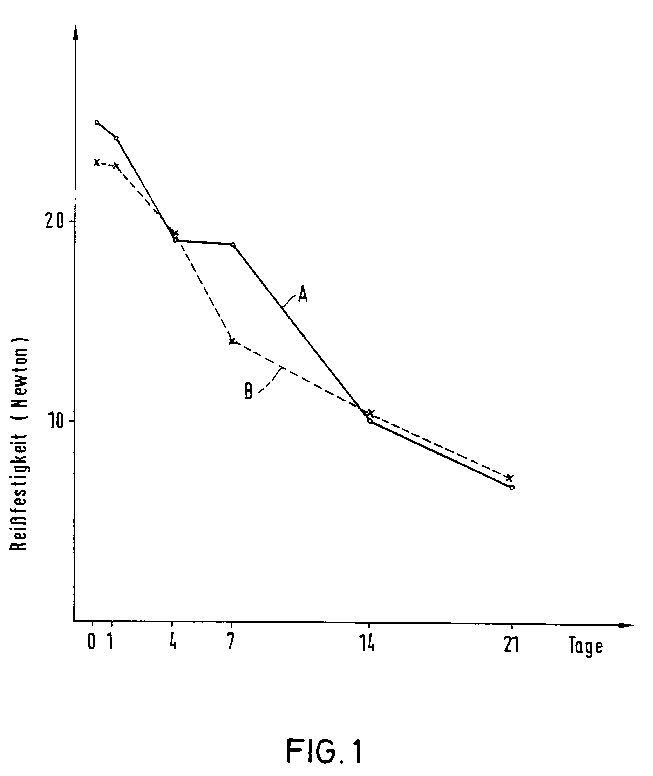

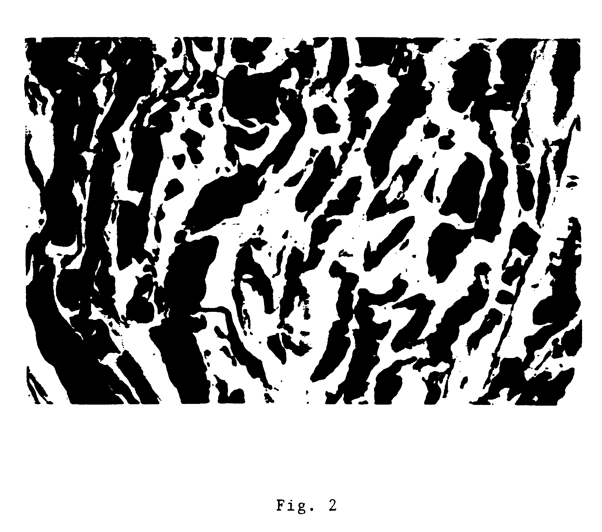

Die Figur 1 zeigt beispielhaft, welche Wirkung durch das erfindungsgemäße Verfahren erzielt werden kann.

Die Figur 1 zeigt den Verlauf der Reißfestigkeit von 10 mm breiten Streifen des erfindungsgemäß erhaltenen Pericard-Materials und im Vergleich zu dem gängigen Marktprodukt Lyodura^{R} nach Einpflanzung unter die Rückenhaut von Ratten (Werte für jeweils 10 Testtierte gemittelt). Hierbei wurden je 50 solcher Streifen implantiert und zu verschiedenen Zeitpunkten nach der Operation entnommen, um dann noch die vorhandene Reißfestigkeit zu messen. Die Kurve A wurde mit Pericard ermittelt, das nach dem Verfahren der Erfindung hergestellt worden war. Die Kurve B wurde zum Vergleich mit Lyodura^{R} ermittelt.

Es kann über den Beobachtungszeitraum von 21 Tagen zwischen dem heterologen, erfindungsgemäß hergestellten Material Pericard und dem homologen Material Lyodura^{R} kein signifikanter Unterschied bezüglich der Reißfestigkeit gefunden werden, womit gezeigt wurde, daß das heterologe Material ein hervorragender Ersatz für das homologe Material darstellt.

**Tabelle**

| Reißfestigkeit von Pericard- und Durastreifen (10 mm breit) nach Implantation (n = 10 pro Gruppe); jeder im Diagramm eingetretene Punkt der Kurve basiert auf 10 Einzelmessungen. | | |
|---|---|---|
| Implantationszeit (Tage) | Pericard gemäß Erfindung (Newton) | Lyodura^{R} (Newton) |
| 0 | 25.0 ± 9.4 | 23 ± 10.0 |
| | 100 % ± 38 | 92 % ± 40 |
| 1 | 24.1 ± 7.9 | 22.8 ± 9.1 |
| | 96 % ± 32 | 91 % ± 36 |
| 4 | 19.0 ± 9.0 | 19.2 ± 8.5 |
| | 76 % ± 36 | 77 % ± 34 |
| 7 | 18.8 ± 6.3 | 14 ± 5.1 |
| | 75 % ± 25 | 56 % ± 20 |
| 14 | 10.0 ± 4.5 | 10.1 ± 4.5 |
| | 40 % ± 18 | 40 % ± 18 |
| 21 | 6.5 ± 3.0 | 7.0 ± 4.5 |
| | 26 % ± 12 | 28 % ± 18 |
| zweite Zeile = Angabe in % O-Wert Pericard = 100 % | | |

### Beurteilung der Biokompatibilität im subkutanen Implantationstest

I. An insgesamt 58 Kaninchen und 220 Ratten wurden subkutane Implantationen von Pericard vorgenommen. Untersuchungsparameter waren der pathologisch-anatomische Befund und die histologische Untersuchung der Implantate nach abgestuften Zeitpunkten von 7 Tagen bis zu 12 Monaten.

Die erfindungsgemäßen Pericardimplantate wurden mit lyophilisierter Dura, Lyodura^{R} sowie mit acetongetrockneter Dura mater (Tutoplast^{R}) verglichen. Es wurden makroskopisch die Entzündungsreaktion und die Implantatbeschaffenheit beurteilt. Anschließend wurden die Proben mit 4 %igem Formalin fixiert und einer histologischen Untersuchung unterzogen.

Die Mikroarchitektur des Kollagengerüstes wurde mit nicht implantierten Kontrollen verglichen.

Die histologische Beurteilung der Implantate erstreckt sich auf das Invasionsverhalten von Zellen (Bindegewebszellen, immunokompetente Zellen), die Verbindung Implantat-Empfänger (Einwachsverhalten), Resorption bzw. Vitalisierung des Fremdmaterials und mögliche Abstoßungsreaktionen.

### Ergebnisse:

Bei der Untersuchung der devitalisierten Pericardproben vor der Implantation wurde nachgewiesen, daß diese vollkommen frei von Zellen oder Resten von Zellbestandteilen waren. Dagegen wiesen lyophilisierte und insbesondere lösungsmittelgetrocknete Dura mater noch vereinzelt Kernmaterial auf.
a) Mikroarchitektur der Implantate:
   Es konnte festgestellt werden, daß die Anordnung der Kollagenfaserstruktur wichtig ist für das Einwanderungsverhalten von Bindegewebszellen.
   Straffe, dicht liegende Kollagenfaserbündel (bei lösungsmittelgetrockneter Dura) behindern die Revitalisierung. Bei dem meist als dissoziierte Fasern vorliegenden Pericard und der Lyodura^{R} ist die Repopulation mit Zellen aus der Bindegewebsreihe erleichtert.
b) Vitalität des Implantates:
   Das Pericard-Implantat verhielt sich als Leitschiene, in dessen Zwischenräume die Zellen einwandern:
   Bereits nach einer Woche sind in das Implantat reichlich Zellen eingewandert, die sich diffus ausgebreitet haben. Es handelt sich um Fibroblasten und histiozytäre Elemente. Der Vitalitätsindex nimmt von der ersten bis zur 6. Woche hin zu.
c) Infiltration des Implantates mit nicht zur Bindegewebsreihe gehörenden Zellen wie Lymphozyten, Makrophagen, Fremdkörperriesenzellen.
   Das Auftreten dieser Zellen, die quantitativ erstaunlicherweise in geringerer Zahl bei den Pericardproben auftraten als bei den Duraproben, ist nicht mit einer aufkommenden Abstoßungsreaktion gleichzusetzen, sondern als immuninformativer Vorgang aufzufassen. Im Gegensatz zur Invasion mit Fibroblasten und Histiozyten dringen diese Lymphozyten, Makrophagen und Riesenzellen selten tief in das Implantat ein. Makrophagen traten gehäuft auf, wo es zu Blutungen ins Wundbett kam. Die Zellen (Ly., Makr., RZ) wurden ausgewählt und ein Index berechnet, der bei der zum klinischen Einsatz kommenden Pericardprobe (1,43) im Vergleich zu den Duraproben (lyophilisiert: 2,2; acetongetrocknet: 2,32) niedriger lag. Dieses Phänomen ist wahrscheinlich darauf zurückzuführen, daß Pericard sehr gut devitalisiert ist, und keine Zell- bzw. Zellkernreste mehr enthält, die eventuell immunogen wirken könnten.
d) Akute Entzündungs- und Abstoßreaktionen
   Bei der Pericardprobe wurden keine Immunoreaktionen nachgewiesen.

Zusammenfassend konnte festgestellt werden, daß das bessere Abschneiden der Pericardproben nicht nur auf das Ausbleiben von Abstoßungsreaktionen zurückzuführen ist, sondern auch auf den besseren Vitalitätsaspekt, wobei hier die etwas geringere Dicke der Pericardproben eine Rolle spielt.

II. Beurteilung von Biokompatibilität und Funktionsfähigkeit von Pericard als Dura mater cerebralis-Ersatz beim Hund
Nach 3 und 6 Monaten post implantationem war das Implantat so gut integriert, daß es von autochthoner Dura nicht mehr zu unterscheiden war (von Fibrozyten revitalisiert und in den Randzonen von Blutgefäßen durchzogen). Die Innenseite des Implantates ist vom gleichen Zelltyp überzogen wie autologe Dura. Verwachsungen zur Gehirnrinde bestanden nicht.

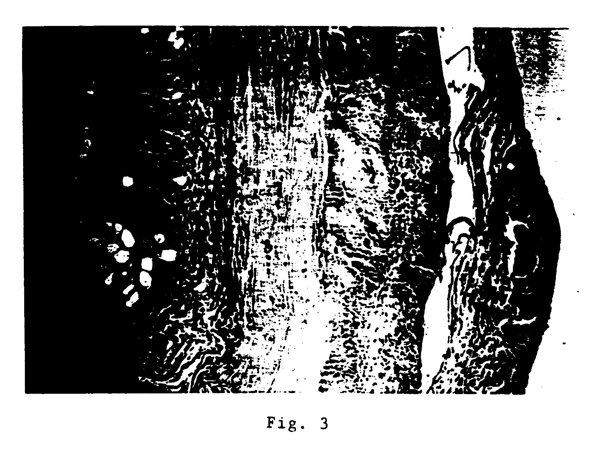

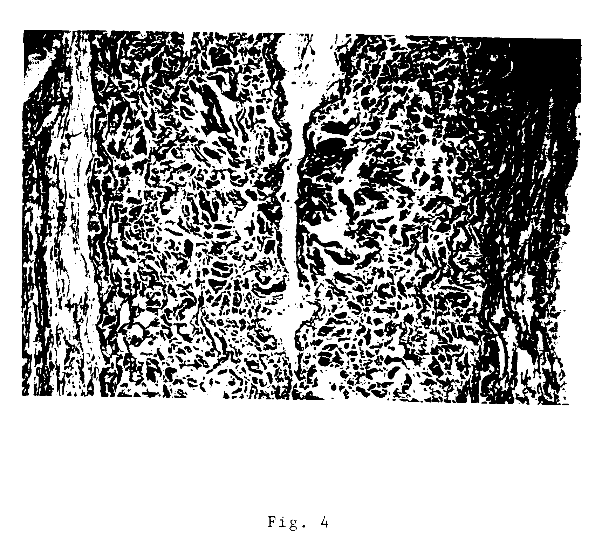

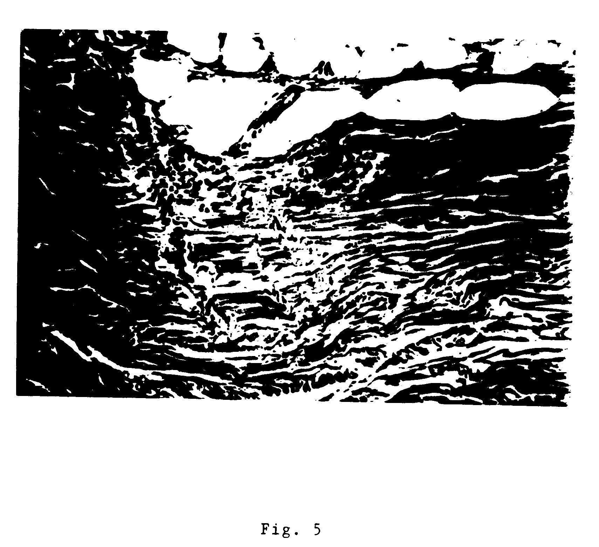

Dieser Effekt des mittels des erfindungsgemäßen Verfahrens hergestellten Rinder-Pericard-Materials im Vergleich zu handelsübichen Dura-Materialien wird in den Figuren 2 - 5 dokumentiert.

Es zeigen:
- Figur 2:: Pericard 6 Wochen nach subkutaner Implantation bei der Ratte. Gut revitalisiertes, vollkommen reizloses Implantat mit vitalen Fibroblasten ohne Lymphozyten, Neubildung von Kollagen.
- Figur 3:: Lösungsmittelgetrocknete Dura mater 6 Wochen nach subkutaner Implantation bei der Ratte (Vergleich). Lokale Akkumulation von Lymphozyten, im zentralen Bereich noch keine Revitalisierung durch Fibroblasten.
- Figur 4:: Pericard 12 Wochen nach subkutaner Implantation bei der Ratte. Gute Integration von Implantat und Wirtsgewebe, keine immunokompetenten Zellen vorhanden.
- Figur 5:: Lösungsmittelgetrocknete Dura mater 12 Wochen p.i. (Vergleich). Lokale Akkumulation von Lymphozyten; das Transplantat ist durch die dichte Fasterstruktur weitgehend avital.

Das erfindungsgemäße Verfahren wird nachstehend anhand eines Ausführungsbeispiels zur Herstellung von Rinder-Pericard erläutert.

### Ausführungsbeispiel

### 1. Rohstoff-Gewinnung

Die als Ausgangsmaterial dienenden Herzbeutel (Pericardium) von Rindern werden im Schlachthof nach der üblichen Fleischbeschau durch einen amtlichen Tierarzt zunächst von anhaftenden Organteilen abtetrennt und grob von Fett- und Bindegewebe befreit. Man erhält auf diese Weise flächige Stücke von der ungefähren Größe 30 x 15 cm und einem Stückgewicht von etwa 1 kg. Diese so vorbereiteten Rinderherzbeutel werden dann in einer mit Eis beschickten Kühltasche vom Schlachthof zur Produktionsstätte transportiert und dort je nach Menge des anfallenden Rohmaterials vor der Weiterverarbeitung bei unter -20 °C zwischengelagert.

### 2. Naßchemische Aufbereitung

Die rohen Pericard-Stücke werden zunächst einzeln mit gereinigtem Wasser gespült - üblicherweise fließend gewässert - um anhaftendes Blut und wasserlösliche Eiweißanteile zu entfernen.

Nach dem Wässern werden alle makroskopisch sichtbaren Reste von Fettgewebe und Basalmembranen entfernt. Anschließend erfolgt eine Behandlung mit 2 %iger wäßriger Natronlauge bei Raumtemperatur. Die Pericardstücke (5000 g) verbleiben insgesamt 16 Stunden im Laugenbad (37,5 l). Nach Entnahme erfolgt dann ein ca. 10 Minuten dauernder Spülprozeß in entmineralisiertem Wasser, der sooft wiederholt wird, bis der pH des Spülwassers auf einen Wert unter 8 gesunken ist. Dies ist nach ca. 1 Stunde erreicht. Sollten noch Basalmembranen und Fettreste festzustellen sein, so werden sie auf dieser Prozeßstufe endgültig entfernt.

Die stark gequollenen Pericard-Stücke werden nun in 37,5 l einer 10 %igen wäßrigen Kochsalzlösung transferiert, um den für die weiteren Prozeßschritte erforderlichen Quellzustand (partielle Entquellung) einzustellen. Die NaCl-Behandlung wird bei Raumtemperatur durchgeführt. Es schließt sich ein Spülprozeß mit entmineralisiertem Wasser an.

Um störende Schwermetallionen und etwaige Kalkeinlagerungen aus dem Pericardgewebe zu entfernen, erfolgt nun eine Behandlung mit 37,5 l schwach alkalisch eingestellter EDTA-Lösung, Zusammensetzung pro 100 ml: 0,3 g. Anschließend wird wie in den vorausgegangenen Prozeßschritten mit entmineralisiertem Wasser gespült, um überschüssigen Komplexbildner zu entfernen und gleichzeitig den pH auf einen Wert von 8,5 zu bringen. Die nun folgende Behandlung mit 1 mal 37,5 l Acetat-Puffer (pH = 4,8, Zusammensetzung pro 100 ml: 0,01 mol/100 ml Na-acetat . 3 H₂O, 59 Volumen-Teile; 0,01 mol/100 ml Essigsäure, 41 Volumen-Teile) dient dem Zweck, alle etwa noch im Pericard-Gewebe verbliebenen Laugenreste abzupuffern und ein schwach saures Milieu für den späteren Bleichprozeß vorzubereiten. Überschüssige Puffersubstanzen werden wie vorstehend beschrieben durch Spülen mit entmineralisiertem Wasser entfernt.

### 3. Oxidative Bleichung

Im Anschluß an die naßchemische Aufbereitung werden die Pericard-Stücke einer einstündigen oxidativen Bleichung in 37,5 l 1,5 %iger Wasserstoffperoxid-Lösung unterzogen. Wie alle vorausgegangenen Behandlungsschritte wird auch der Bleichprozeß bei Raumtemperatur durchgeführt. Auf diese Weise wird einerseits die Effizienz der Reinigungsoperationen sichergestellt, andererseits aber eine Beeinträchtigung des kollagenen Gewebes vermieden.

### 4. Auswaschen

Zur Entfernung überschüssigen Reagenzes wird anschließend mit entmineralisiertem Wasser gemäß dem üblichen Programm gespült.

### 5. Entfettung

Die gespülten Rinder-Pericard-Stücke wurden in eine solche Menge Aceton gelegt, daß das Rinder-Pericard-Gewebe vollständig mit Aceton überdeckt war.

Innerhalb von 8 Stunden wurde das Lösungsmittel dreimal erneuert. Die so entwässerten Rinder-Pericard-Gewebe wurden daraufhin in eine Soxhlet-Apparatur überführt und ca. 7 Stunden mit Aceton extrahiert. Nach der Extraktion wurden die Pericard-Stücke an der Luft getrocknet und anschließend in einem Transport-Gefäß mit entmineralisiertem Wasser rehydratisiert.

### 6. Gefriertrocknung

Die Trocknung erfolgte in einer automatisch gesteuerten Gefriertrocknungsanlage. Die Gefriertrocknung verläuft im einzelnen wie folgt:
Temperaturabsenkung auf +1 °C, Temperaturabsenkung auf -40 °C, Einschalten des Vakuums, Aufheizung der Stellplatten auf +40 °C und Trocknung bei vollem Vakuum.

### 7. Sterilisieren

Die Sterilisierung erfolgt durch Strahlensterilisation mit 2,5 Mrad.

## Patentansprüche

1. Verfahren zur Herstellung von Rinder-Pericard-Material durch Aufarbeitung von rohem Rinder-Pericard-Gewebe
durch eine naßchemische Aufbereitung, oxidative Bleichung, Auswaschen, Entfetten, Trocknen und Sterilisieren,
dadurch gekennzeichnet, daß die naßchemische Aufbereitung des Rinder-Pericard-Gewebes folgende Schritte umfaßt:
a) Wässern des Rinder-Pericard-Gewebes,
b) mechanische Entfernung von Fettgewebe und Basalmembranen von der Oberfläche des Rinder-Pericard-Gewebes,
c) Behandlung des Rinder-Pericard-Gewebes mit einer verdünnten, wäßrigen, basischen Lösung von Natriumhydroxid, Kaliumhydroxid, Lithiumhydroxid und/oder Natriumcarbonat über einen Zeitraum von bis zu 16 Stunden,
d) Spülen des Rinder-Pericard-Gewebes mit entmineralisiertem Wasser zur Entfernung von restlicher Base,
e) Behandlung des Rinder-Pericard-Gewebes mit verdünnter wäßriger Natriumchloridlösung,
f) Spülen des Rinder-Pericard-Gewebes mit entmineralisiertem Wasser zur Entfernung von Natriumchloridresten,
g) Behandlung des Rinder-Pericard-Gewebes mit einem Komplexierungsmittel,
h) Spülen des Rinder-Pericard-Gewebes mit entmineralisiertem Wasser zur Entfernung von Komplexierungsmittelresten,
i) Behandlung des Rinder-Pericard-Gewebes mit einem sauren Puffersystem und
j) Spülen des Rinder-Pericard-Gewebes mit entmineralisiertem Wasser zur Entfernung von sauren Puffersystemresten, und daß man das Entfetten und Trocknen durch Aceton-Behandlung bei Raumtemperatur, Soxhlet-Extraktion mit Aceton, Rehydratisierung und Gefriertrockung durchführt.

2. Verfahren nach Anspruch 1, dadurch gekennzeichnet, daß man im Anschluß an Verfahrensschritt d) vor Verfahrensschritt e) die Maßnahme nach Merkmal b) wiederholt.

3. Verfahren nach Anspruch 1 oder 2, dadurch gekennzeichnet, daß die naßchemischen Aufbereitungsschritte a) und c) bis j) bei Raumtemperatur, also zwischen 15 °C und 25 °C, durchgeführt werden.

4. Verfahren nach Ansprüchen 1 bis 3, dadurch gekennzeichnet, daß man für die Behandlung im Verfahrensschritt c) eine 2 bis 2,5 Gew.-%ige Natriumhydroxidlösung verwendet.

5. Verfahren nach Ansprüchen 1 bis 3, dadurch gekennzeichnet, daß man die Behandlung in Verfahrensschritt e) mit einer wäßrigen, 10 bis 11 Gew.-%igen Natriumchloridlösung, durchführt.

6. Verfahren nach Ansprüchen 1 bis 3, dadurch gekennzeichnet, daß man die Behandlung in Verfahrensschritt g) mit einer verdünnten wäßrigen Dinatrium-EDTA-Lösung, welche vorzugsweise 0,3 bis 0,5 Gew.-%ig ist, in einem alkalisch-wäßrigen Medium (pH ≧ 11) durchführt.

7. Verfahren nach Ansprüchen 1 bis 3, dadurch gekennzeichnet, daß man die Spülung in Verfahrensschritt h) solange fortsetzt, bis man einen schwach-alkalischen pH-Wert von bis zu 8,5 erreicht hat.

8. Verfahren nach Ansprüchen 1 bis 3, dadurch gekennzeichnet, daß man die Behandlung in Verfahrensschritt i) mit einem Acetat-Puffersystem mit einem pH-Wert von 4,5 bis 6, vorzugsweise 4,8 ausführt.

9. Verfahren nach Ansprüchen 1 bis 8, dadurch gekennzeichnet, daß man die oxidative Bleichung mit Wasserstoffperoxid enthaltenden wäßrigen Lösungen mit einem Gehalt von weniger als 2 Gew.-%, vorzugsweise um 1,5 Gew.-% vornimmt.

## Claims

1. A process for preparing bovine pericard material by subjecting raw bovine pericard tissue to operations of wet-chemical processing, oxidative bleaching, washing out, de-greasing, drying and sterilizing,
characterized in that the wet-chemical processing of the bovine pericard tissue comprises the following steps:
a) Soaking the bovine pericard tissue;
b) Mechanically removing fat tissue and basal membranes from the surface of the bovine pericard tissue;
c) Treating the bovine pericard tissue with a diluted aqueous basic solution of sodium hydroxide, potassium hydroxide, lithium hydroxide and/or sodium carbonate over a period of up to 16 hours;
d) Rinsing the bovine pericard tissue with demineralized water to remove residual base;
e) Treating the bovine pericard tissue with a diluted aqueous sodium chloride solution;
f) Rinsing the bovine pericard tissue with demineralized water to remove residual sodium chloride;
g) Treating the bovine pericard tissue with a complexing agent;
h) Rinsing the bovine pericard tissue with demineralized water to remove residual complexing agent;
i) Treating the bovine pericard tissue with an acidic buffer system; and
j) Rinsing the bovine pericard tissue with de-mineralized water to remove residual acidic buffer system, and
that de-greasing and drying are carried out by means of an acetone treatment at room temperature, Soxhlet extraction with acetone, re-hydration and freeze drying.

2. The process according to claim 1, characterized in that the measure according to feature b) is repeated subsequently to process step d) prior to process step e).

3. The process according to claims 1 or 2, characterized in that the wet-chemical processing steps a) and c) through j) are conducted at room temperature, that is between 15 °C and 25 °C.

4. The process according to claims 1 to 3, characterized in that for the treatment in process step c) a 2 to 2.5% by weight sodium hydroxide solution is used.

5. The process according to claims 1 to 3, characterized in that the treatment in process step e) is carried out with an aqueous 10 to 11% by weight sodium chloride solution.

6. The process according to claims 1 to 3, characterized in that the treatment in process step g) is carried out with a diluted aqueous disodium EDTA solution which preferably is 0.3 to 0.5% by weight in an aqueous alkaline medium pH ≧ 11).

7. The process according to claims 1 to 3, characterized in that the rinsing procedure in process step h) is continued until a weakly alkaline pH value of up to 8.5 has been reached.

8. The process according to claims 1 to 3, characterized in that the treatment in process step i) is carried out with an acetate buffer system at a pH value of from 4.5 to 6, and preferably of 4.8.

9. The process according to claims 1 to 8, characterized in that the oxidative bleaching is effected with aqueous solutions containing hydrogen peroxide at a content of less than 2% by weight, and preferably of about 1.5% by weight.

## Revendications

1. Procédé de production d'une matière de péricarde de bovin, par traitement de la matière brute de tissu de péricarde de bovin par voie chimique au mouillé, blanchiment oxydant, lavage, dégraissage, séchage et stérilisation, procédé caractérisé en ce que le traitement chimique au mouillé du tissu de péricarde de bovin comprend les étapes suivantes :
a) passage à l'eau du tissu de péricarde de bovin;
b) enlèvement mécanique de tissu gras et de membranes de base de la surface du tissu de péricarde bovin;
c) traitement du tissu de péricarde de bovin par une solution aqueuse basique diluée d'hydroxyde de sodium, d'hydroxyde de potassium, d'hydroxyde de lithium et/ou de carbonate de sodium en un espace de temps allant jusqu'à 16 heures,
d) rinçage à l'eau déminéralisée du tissu de péricarde de bovin pour enlever la base restante,
e) traitement du tissu de péricarde de bovin par une solution aqueuse diluée de chlorure de sodium,
f) rinçage du tissu de péricarde de bovin par de l'eau déminéralisée, pour enlever les restes de chlorure de sodium,
g) traitement du tissu de péricarde de bovin par un agent complexant,
h) rinçage à l'eau déminéralisée du tissu de péricarde de bovin pour enlever les restes de l'agent complexant,
i) traitement, par un système tampon acide, du tissu de péricarde de bovin, et
j) rinçage à l'eau déminéralisée du tissu de péricarde de bovin pour enlever les restes du système tampon acide, et en ce que l'on effectue le dégraissage et le séchage par traitement à l'acétone à la température ambiante, par extraction à l'acétone à l'aide d'un appareil Soxhlet, réhydratation et lyophilisation.

2. Procédé selon la revendication 1, caractérisé en ce qu'après l'étape opératoire (d) et avant l'étape opératoire (e), on répète la mesure appliquée selon l'étape ou particularité (b).

3. Procédé selon la revendication 1 ou 2, caractérisé en ce qu'on effectue les étapes de préparation chimique au mouillé (a) et (c) à (j) à la température ambiante, donc entre 15°C et 25°C.

4. Procédé selon les revendications 1 à 3 caractérisé en ce que, pour le traitement dans l'étape opératoire (c), on utilise une solution comportant 2 à 2,5 % en poids d'hydroxyde de sodium.

5. Procédé selon les revendications 1 à 3, caractérisé en ce qu'on effectue le traitement, dans l'étape opératoire (e), avec une solution aqueuse contenant 10 à 11% en poids de chlorure de sodium.

6. Procédé selon les revendications 1 à 3, caractérisé en ce qu'on effectue le traitement dans l'étape opératoire (g) avec une solution aqueuse diluée de EDTA disodique (acide éthylène diamine tétraacétique disodique), qui est avantageusement à O,3 jusqu'à O,5 % en poids, dans un milieu alcalin aqueux ( à pH supérieur ou égal à 11).

7. Procédé selon les revendications 1 à 3, caractérisé en ce qu'on poursuit, dans l'étape opératoire (h) le rinçage jusqu'à atteindre une valeur faiblement alcaline du pH allant jusqu'à 8,5.

8. Procédé selon les revendications 1 à 3, caractérisé en ce qu'on effectue le traitement, dans l'étape opératoire (i) avec un système tampon d'acétate ayant une valeur de pH de 4,5 à 6, avantageusement une valeur de pH de 4,8.

9. Procédé selon les revendications 1 à 8, caractérisé en ce qu'on entreprend le blanchiment oxydant à l'aide de solutions aqueuses contenant du peroxyde d'hydrogène et présentant une teneur inférieure à 2% en poids, avantageusement une teneur de 1,5% en poids.
